(19) Europäisches Patentamt
European Patent Office
Office européen des brevets

(11) Veröffentlichungsnummer: **0 088 724**
**B1**

(12) # EUROPÄISCHE PATENTSCHRIFT

(45) Veröffentlichungstag der Patentschrift: **18.05.88**

(51) Int. Cl.[4]: **C 07 D 249/18**, C 10 M 133/44

(21) Anmeldenummer: **83810077.4**

(22) Anmeldetag: **21.02.83**

(54) **Benzotriazolgemische, Verfahren zu deren Herstellung und ihre Verwendung als Metallpassivatoren.**

(30) Priorität: **26.02.82 CH 1198/82**

(43) Veröffentlichungstag der Anmeldung: **14.09.83 Patentblatt 83/37**

(45) Bekanntmachung des Hinweises auf die Patenterteilung: **18.05.88 Patentblatt 88/20**

(84) Benannte Vertragsstaaten: **BE CH DE FR IT LI NL SE**

(56) Entgegenhaltungen:
**DE-A-2 601 719**
**GB-A-1 511 593**
**GB-A-1 514 359**
**US-A-4 107 060**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Regenass, Franz, Dr., Oristalstrasse 45, CH- 4410 Liestal (CH)**
Erfinder: **Hamblin, Peter Collen, Dr., Alemannenweg 12, CH- 4112 Flüh (CH)**

**EP 0 088 724 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue, flüssige Benzotriazol-Gemische, ein Verfahren zu deren Herstellung und ihre Verwendung als Metallpassivatoren für funktionelle Flüssigkeiten.

Es ist bekannt, dass organische Verbindungen als Metallpassivatoren, beispielsweise für Kupfer oder Silber, in funktionellen Substraten wie hydraulischen Flüssigkeiten oder in Frostschutzlösungen zum Schutz von Kupfer- oder Silbergegenständen gegen Korrosion verwendet werden können. Bekannte Verbindungen hierfür sind beispielsweise Benzimidazol-, Benzothiazol- und Benzotriazolderivate.

Die letztgenannten Verbindungen sind beispielsweise in der GB-Patentschrift 1 061 904 beschrieben. Benzotriazol selbst und viele seiner Derivate sind jedoch in ihrer Anwendung wegen ihrer schlechten Löslichkeit, insbesondere in Mineralölen, beschränkt verwendbar.

Besonders vorteilhaft für derartige funktionelle Flüssigkeiten sind Metallpassivatoren, die eine hohe Löslichkeit aufweisen, so dass sie in den Substraten, worin sie eingesetzt werden, rasch und gut verteilt werden können, was einen optimalen Schutz für die Metallgegenstände gewährleistet.

So wurde in der US Patentschrift 4 177 155 bereits vorgeschlagen, die Löslichkeit derartiger Metallpassivatoren, beispielsweise bestimmter Benzotriazolderivate, in funktionellen Flüssigkeiten, sowohl auf Wasser- als duch auf Ölbasis, durch Einführung von molaren Gruppen zu verbessern. Derartige Verbindungen sind jedoch wegen ihres festen, oeligen oder wachsartigen Zustandes nicht optimal. Die oeligen und wachsartigen Produkte erschweren die Einarbeitung bzw. Eindosierung in die Substrate, mit den festen Produkten dagegen ist eine rasche Auflösung nicht möglich, was den Mischvorgang verlangsamt.

Ausserdem entwickelt sich bei der Verwendung bzw. Eindosierung von festen Produkten öfters Staub, was einen weiteren Nachteil darstellt. Es wurden nun, im Sinne einer Auswahl aus der obenerwähnten US Patentschrift 4 177 155, gut lösliche Benzotriazolderivate gefunden.

Gegenstand der Erfindung ist ein flüssiges Gemisch der Verbindungen der Formeln I und II

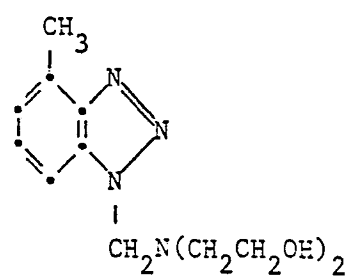

(I)

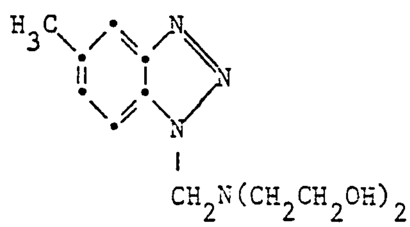

(II)

Bevorzugt ist ein flüssiges Gemisch aus 50 bis 70 Gewichtsprozent der Verbindung der Formel I und 50 bis 30 Gewichtsprozent der Verbindung der Formel II.

Die erfindungsgemässen Gemische stellen Flüssigkeiten dar, die eine ausgezeichnete Löslichkeit in Wasser aufweisen. Sie zeichnen sich durch eine rasche Dispergierbarkeit und gute Verteilung in den funktionellen Flüssigkeiten auf Wasserbasis aus. Es ist ausserdem möglich, sie durch Flüssig-Flüssig-Eindosierung in die Substrate einzuarbeiten.

Auch Gegenstand der Erfindung ist ein Verfahren zur Herstellung von flüssigen Gemischen der Verbindungen der Formeln I und II, dadurch gekennzeichnet, dass man nach an sich bekannter Weise ein Gemisch der Verbindungen der Formeln III und IV

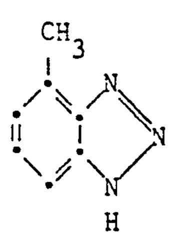

(III)

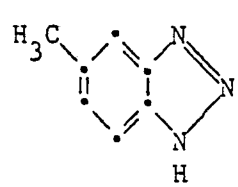

(IV)

mit Formaldehyd und Diäthanolamin umsetzt. Bevorzugt wird ein Gemisch aus 50 bis 70 Gewichtsprozent der Verbindung der Formel III und 50 bis 30 Gewichtsprozent der Formel IV eingesetzt.

Die benötigten Reaktanden der Formeln III und IV bzw. Gemische davon stellen bekannte Verbindungen dar und können beispielsweise nach dem in GB-PS-1 065 995 beschriebenen Verfahren hergestellt werden.

Das Reagenz Formaldehyd wird bevorzugt in der Handelsform verwendet, z. B. als Formalin (wässrige Lösung) oder Paraformaldehyd.

Die molaren Verhältnisse des Gemisches 4- bzw. 5-Methylbenzotriazol zu Formaldehyd und zu Diäthanolamin liegen bevorzugt in etwa äquimolaren Mengen.

Das Verfahren wird insbesondere ausgeführt, indem man alle Reaktanden zusammen auf erhöhte Temperatur erhitzt, z. B. auf 50 120°C. Wenn erwünscht, können Formaldehyd und das entsprechende Gemisch aus 4- und 5-Methylbenzotriazol zunächst miteinander zu den entsprechenden N-Methylolderivaten umgesetzt werden, bevor mit Diäthanolamin weiter reagiert wird.

Das Verfahren wird bevorzugt ohne organische Lösungsmittel durchgeführt.

Es kann aber auch in Anwesenheit eines inerten organischen Lösungsmittels gearbeitet werden. Geeignete Lösungsmittel dafür sind beispielsweise aliphatische und aromatische Kohlenwasserstoffe, aliphatische Alkohole und Ketone, Äthylenglykolmonoäther, Polyäthylenglykolmonoäther, Äthylenglykol oder Propylenglykol.

Eine besondere Ausführungsform des Verfahrens besteht darin, dass das erhaltene Reaktionsgemisch nach Reaktionsbeendigung ohne irgendwelche zusätzliche Reinigung oder allfällige Abtrennung des Lösungsmittels klarfiltriert wird und direkt ins Gebinde zum weiteren Verbrauch eingefüllt wird.

Flüssige Gemische der Verbindungen der Formel I und II können ausserdem auch durch getrennte Synthese der einzelnen Verbindungen der Formeln I resp. II aus den Verbindungen der Formeln III resp. IV und anschliessende Mischung in gewünschtem Verhältnis zueinander hergestellt werden.

Die flüssigen Gemische der Verbindungen der Formeln I und II stellen Mannich-Basen dar, welche auf Grund der Tautomerie des Triazolrestes und der Anwesenheit der Methylgruppe im Benzolkern (Stellungen 4 und 5) in verschiedenen Isomeren existieren können (Formeln V, VI und VII):

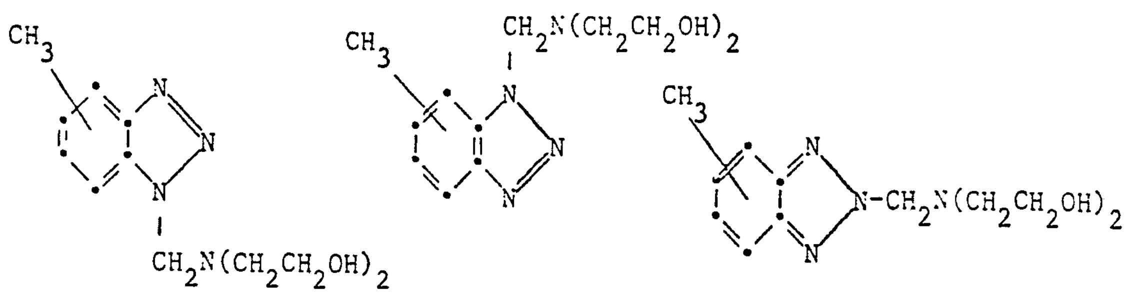

(V) (VI) (VII)

Aus der Fachliteratur, beispielsweise Advances in Heterocyclic Chemistry, by A. Katritzky, The Tautomerism of Heterocycles, Supplement 1, (1976), ist bekannt, dass die sogenannte "1H-Form" in Triazolen überwiegt, weshalb eine dieser isomeren "1H-Formen" V bzw. VI für die Beschreibung der Strukturen I bis IV ausgewählt wurde.

Die flüssigen Gemische der Verbindungen der Formeln I und II stellen bei Raumtemperatur viskose Flüssigkeiten dar und werden deshalb vorzugsweise als wässrige Lösungen eingesetzt.

Derartige Lösungen können durch Zusatz von Wasser in einem Verhältnis Gemisch/$H_2O$ von 99 : 1 bis zu 11 : 89 Gewichtsteilen hergestellt werden, so dass ihre Viskosität je nach Bedarf optimal eingestellt werden kann.

Eine besondere Ausführungsform der Erfindung ist die Verwendung der wässrigen Lösungen enthaltend die Gemische der Verbindungen der Formeln I und II, welche direkt nach der Umsetzung der entsprechenden Methylbenzotriazolgemische mit Diäthanolamin und wässrigem Formaldehyd erhalten werden, insbesondere derjenigen, die zwischen 15 und 30 Gewichtsprozent Wasser enthalten.

Die flüssigen Gemische der Verbindungen der Formeln I und II sind hervorragende Metallpassivatoren mit hoher Löslichkeit, welche in funktionellen Flüssigkeiten eingesetzt werden können.

Beispiele für funktionelle Flüssigkeiten sind hydraulische Flüssigkeiten und Metallbearbeitungs-Flüssigkeiten, für die die erfindungsgemässen Stoffe nützlich sind, auf Basis von wässrigen Polyglykol/Polyglykoläther-Mischungen, Glykol-Systemen, Öl-in-Wasser und Wasser-in-Öl Emulsionen, sowie wässrige Frostschutzmittel auf Glykol-Basis.

Von besonderem Interesse sind Flüssigkeiten, die mit Wasser gemischt werden, z. B. Frostschutzmittel, Hydraulik- und Metallbearbeitungs-Flüssigkeiten.

Je nach der Art der Flüssigkeit enthält die Zusammensetzung insbesondere ein oder mehrere Co-Additive.

Beispiele für Co-Additive für derartige Flüssigkeiten sind Antioxidantien, Korrosions- und Rostinhibitoren, weitere Metallpassivatoren, Hochdruck- und Verschleisschutzmittel (extreme pressure, anti-wear agents), Biocide, Puffermittel und Schaumhemmer.

Beispiele für Antioxidantien sind: 2,6-Ditertiärbutyl-p-kresol und Phenyl-α-naphthylamin.

Beispiele für Korrosions- und Rost-Inhibitoren sind: Natriumnitrit, Natriumbenzoat, Morpholin, Aminseifen, z. B. Triäthanolamin-sebacat, Triäthanolamin-phosphat, Dinatriumhydrogenphosphat, Dinatriumsebacat,

Arylsulphonamido-carbonsäureester.

Beispiele für weitere Metallpassivatoren sind: Benzotriazol und Natriummercaptobenzothiazol. Beispiele für Hochdruck- und Verschleisschutzmittel sind: chloriertes Paraffin, geschwefeltes Samenöl, geschwefelte Olefine, äthoxylierte Halbester und Polyglykole.

Beispiele für Puffer sind: Borax und Triäthanolamin.

Beispiele für Biocide sind: 2,4,5-Trichlorphenol, Natriumsalz von 2,2'-Dihydroxy-5,5'-dichlordiphenyl-methan, und Natriumsalz von Orthophenylphenol. Beispiele für Schaumhemmer sind Silicone und Polymethacrylate.

Die flüssigen Gemische der Verbindungen der Formeln I und II werden den obigen funktionellen Flüssigkeiten in einer Konzentration von 0,001 bis zu 5 Gewichtsprozent, bezogen auf das Gesamtgewicht der Flüssigkeit zugesetzt.

Die erfindungsgemässen flüssigen Gemische der Verbindungen der Formeln I und II und insbesondere die wässrigen Lösungen davon lassen sich durch einfache Flüssig-Flüssig-Eindosierung in hydraulische Flüssigkeiten, Metallbearbeitungsflüssigkeiten und Frostschutzmittel leicht einarbeiten und rasch auflösen, insbesondere bei ihrer Einarbeitung in die Konzentrate derartiger funktionellen Flüssigkeiten, ohne dass dabei ein Erhitzen notwendig ist. Dies führt zu einer wünschenswerten Zeit- und Energieersparnis.

Ein weiterer Vorteil solcher flüssiger Gemische liegt in ihrer besseren Handhabung im Vergleich mit den festen Produkten. Sie können nämlich im gewünschten Mengenverhältnis in die funktionellen flüssigen Substrate gepumpt werden. Die gewünschte Menge kann entweder durch Wägen oder insbesondere durch Messung des Volumens bestimmt werden.

Ein weiterer Vorteil der erfindungsgemässen flüssigen Gemische und Lösungen liegt darin, dass sie im Gegensatz zu vielen festen Produkten keine Staubentwicklung bei der Eindosierung bzw. Einarbeitung verursachen, was in gewerbehygienischer Sicht besonders vorteilhaft ist.

Die folgenden Beispiele illustrieren die Erfindung weiter.

**Beispiel 1**

27,85 Gewichtsteile Diäthanolamin und 35,25 Gewichtsteile eines Gemisches bestehend aus etwa 65 Gewichtsprozent 4-Methylbenzotriazol und 35 Gewichtsprozent 5-Methylbenzotriazol werden gemischt und auf eine Temperatur von 80-85°C geheizt, wobei eine klare Lösung entsteht. Nach Abkühlung dieser Lösung auf eine Temperatur von 70° bis 75°C werden innert ca. einer Stunde und bei dieser Temperatur 21,5 Gewichtsteile Formaldehyd als 37 %-ige wässrige Lösung zugegeben. Dabei wird die Temperatur durch leichte Wasserkühlung gehalten. Die Mischung wird 1 Stunde bei einer Temperatur von 70-75°C nachgerührt. Danach wird die so erhaltene Lösung ohne Abkühlung klarfiltriert und direkt ins Gebinde eingefüllt. Man erhält 84,6 Teile einer Lösung, enthaltend ein Gemisch der Verbindungen der Formeln I und II im entsprechenden Verhältnis 65 : 35 (Gewichtsprozent) zueinander, und 18,4 Gewichtsteile Wasser, in einer praktisch quantitativen Ausbeute. Die Zusammensetzung dieser Lösung besteht aus etwa 78 Gewichtsprozent des Gemisches der Verbindungen der Formeln I und II, etwa 22 Gewichtsprozent Wasser. Die kinematische Viskosität dieser Lösung beträgt 45 $mm^2/sec$ (cSt) bei 40°C.

Die so erhaltene Lösung kann direkt ohne irgendwelche Zwischenisolierung bzw. Reinigung als Metallpassivator in funktionellen Flüssigkeiten verwendet werden. Dank ihrer günstigen Viskosität lässt sie sich in derartige Substrate mittels Flüssig-Flüssig-Eindosierung auf praktische Art und Weise einarbeiten und rasch einmischen bzw. emulgieren.

**Beispiel 2**

Führt man die gleiche Reaktion gemäss Beispeil 1 durch, destilliert man aber das Wasser aus dem Reaktionsgemisch nach Reaktionsbeendigung unter reduziertem Druck ab, so werden 66,2 Teile eines Gemisches der Verbindungen der Formeln I und II in praktisch quantitativer Ausbeute isoliert. Die so erhaltene, hellbraune Flüssigkeit hat folgende Verbrennungsanalyse:

| | | | |
|---|---|---|---|
| Berechnet: | C% | 57,6 | Gefunden: 57,3 |
| | H% | 7,2 | 7,1 |
| | N% | 22,4 | 22,1 |

Sie enthält etwa 65 Gewichtsprozent der Verbindung der Formel I und etwa 35 Gewichtsprozent der Verbindung der Formel II. Diese Flüssigkeit kann als Metallpassivator in funktionellen Flüssigkeiten auch direkt oder aber vorzugsweise nach Verdünnung mit Wasser verwendet werden.

**Beispiel 3**

Proben von Metallen, wie sie typisch in Kühlsystemen von Verbrennungsmaschinen anzutreffen sind, beispielsweise in Autokühlsystemen, werden ganz in eine belüftete Testlösung zwei Wochen (336 Stunden) bei 82°C eingetaucht, gemäss ASTM-Methode D 1384-70. Die Korrosionsschutzwirkung der Lösung wird aufgrund der Gewichtsänderung der Metallproben bestimmt.

Als Testlösung wird die folgende Formulierung verwendet:

67 Gew.-% Wasser
33 Gew.-% Äthylenglykol-Formulierung, bestehend aus
- 92,74 resp. 92,87 Gew.-% Äthylenglykol
- 2,9 Gew.-% Triäthanolamin
- 1,1 Gew.-% Phosphorsäure
- 3,0 Gew.-% Burax und
- 0,26 resp. 0,13 Gew.-% Metallpassivator gemäss Beispiel 1.

Die Ergebnisse sind in der Tabelle 1 zusammengefasst.

| Metallpassivator | Gewichtsänderungen bei Metall, ausgedrückt in mg/Prüfkörperfläche | | | | | |
|---|---|---|---|---|---|---|
| (Gewichtsprozent) | Cu | Messing | Löt-metall | Stahl | Gusseisen | Al |
| Gemisch gemäss Beispiel 1 (0,26 % à 78 %, ≅ 0,2 % à 100 %) | -0,3 | +3,4 | -0,9 | -0,4 | -1,8 | -6,0 |
| Gemisch gemäss Beispiel 1 (0,13 % à 78 %, ≅ 0,1 % à 100 %) | -0,9 | +3,9 | -1,3 | -0,4 | -3,2 | -5,5 |

Die Gewichtsabnahme wird mit dem Vorzeichen -, die Gewichtszunahme mit dem Zeichen + charakterisiert.

**Patentansprüche**

1. Flüssiges Gemisch Verbindungen der Formeln I und II

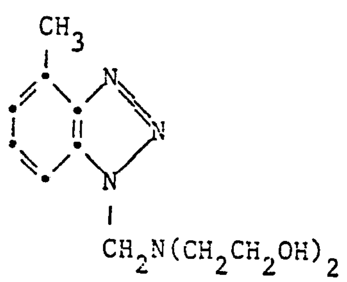

(I)

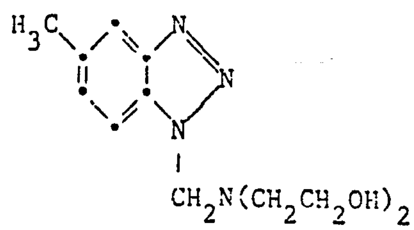

(II)

2. Gemisch nach Anspruch 1, bestehend aus 50 bis 70 Gewichtsprozent der Verbindung der Formel I und 50 bis 30 Gewichtsprozent der Verbindung der Formel II.

3. Gemisch nach Anspruch 1, bestehend aus etwa 65 Gewichtsprozent der Verbindung der Formel I und etwa 35 Gewichtsprozent der Verbindung der Formel II.

4. Gemisch nach Anspruch 1 in Form einer wässrigen Lösung.

5. Verbindung der Formel II gemäss Anspruch 1.

6. Verfahren zur Herstellung eines Gemisches gemäss Anspruch 1, dadurch gekennzeichnet, dass ein Gemisch der Verbindungen der Formeln III und IV

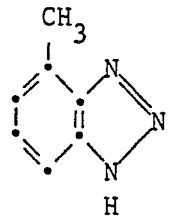

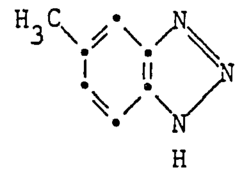

(III) (IV)

mit Formaldehyd und Diäthanolamin umgesetzt wird.

7. Verfahren nach Anspruch 6, wobei die Reaktanden, nämlich Gemisch der Verbindungen der Formeln III und IV, Formaldehyd und Diäthanolamin in etwa molarem Verhältnis eingesetzt werden.

8. Vefahren nach Anspruch 6, wobei Formaldehyd in Form von Formalin eingesetzt wird.

9. Funktionelle Flüssigkeit, enthaltend ein Gemisch gemäss Anspruch 1.

10. Funktionelle Flüssigkeit nach Anspruch 9, wobei das Gemisch der Verbindungen der Formeln I und II 0,001 bis 5 Gewichtsprozent des Gesamtgewichts der Flüssigkeit ausmacht.

11. Als funktionelle Flüssigkeit gemäss Anspruch 9 eine hydraulische Flüssigkeit oder eine Metallbearbeitungs-Flüssigkeit auf Basis von wässrigen Polyglykol-/Polyglykoläther-Mischungen, wässrigen Glykolsystemen, Öl-in-Wasser oder Wasser-in-Öl Emulsionen, oder wassermischbares Frostschutzmittel auf Glykolbasis.

12. Funktionelle Flüssigkeit nach Anspruch 9, worin ein oder mehrere Co-Additive vorhanden sind.

**Claims**

1. A liquid mixture of the compounds of formulae I and II

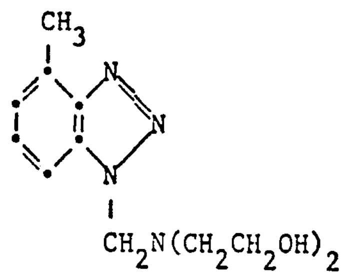

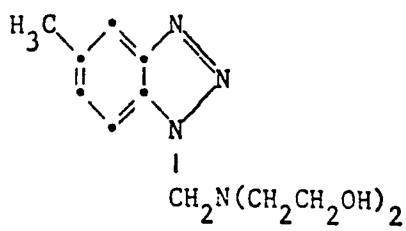

(I) (II)

2. A mixture according to claim 1 comprising 50 to 70 per cent by weight of the compound of formula I and 50 to 30 per cent by weight of the compound of formula II.

3. A mixture according to claim 1 comprising about 65 per cent by weight of the compound of formula I and about 35 per cent by weight of the compound of formula II.

4. A mixture according to claim 1 in the form of an aqueous solution.

5. A compound of formula II according to claim 1.

6. A process for the preparation of a mixture according to claim 1, which process comprises reacting a mixture of the compounds of formulae III and IV

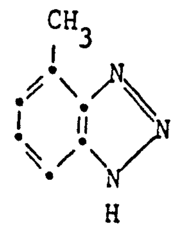

(III)

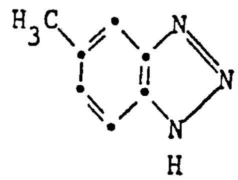

(IV)

with formaldehyde and diethanolamine.

7. A process according to claim 6, wherein the reactants, namely a mixture of the compounds of formulae III and IV, formaldehyde and diethanolamine, are used in approximately molar ratio.

8. A process according to claim 6, wherein formaldehyde is used in the form of formalin.

9. A functional fluid containing a mixture according to claim 1.

10. A functional fluid according to claim 9, wherein the mixture of the compounds of formulae I and II constitutes 0.001 to 5 per cent by weight of the total weight of the fluid.

11. As functional fluid according to claim 9, a hydraulic fluid or a metal-working fluid based on aqueous polyglycol/polyglycol ether mixtures, aqueous glycol systems, oil-in-water or water-in-oil emulsions, or water-miscible anti-freeze compositions based on glycol.

12. A functional fluid according to claim 9, wherein one or more co-additives are present.

**Revendications**

1. Mélange liquide des composés de formules I et II:

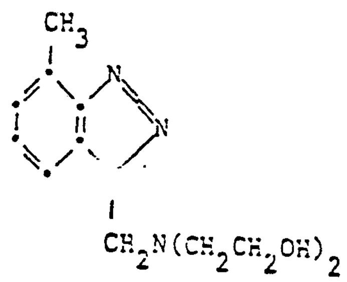

(I)

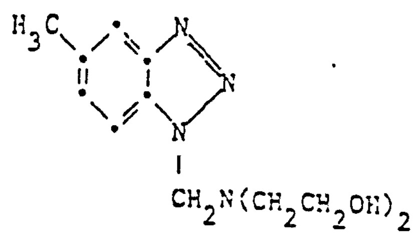

(II)

2. Mélange selon la revendication 1 qui est constitué de 50 à 70 % en poids du composé de formule 1 et de 50 à 30 % en poids du composé de formule II.

3. Mélange selon la revendication 1 qui est constitué d'environ 65 % en poids du composé de formule I et d'environ 35 % en poids du composé de formule II.

4. Mélange selon la revendication 1 sous la forme d'une solution aqueuse.

5. Composé de formule II selon la revendication 1.

6. Procédé pour préparer un mélange selon la revendication 1, procédé caractérisé en ce qu'on fait réagir un mélange des composés de formules III et IV:

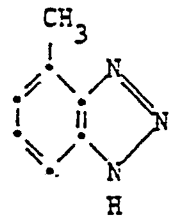

(III)

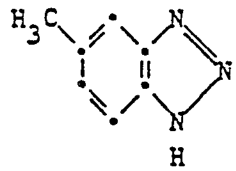

(IV)

avec le formaldéhyde et la diéthanolamine.

7. Procédé selon la revendication 6 caractérisé en ce que les réactants, c'est-à-dire le mélange des composés de formules III et IV, le formaldéhyde et la diéthanolamine, sont mis en jeu en des proportions à peu près équimolaires.

8. Procédé selon la revendication 6 dans lequel le formaldéhyde est mis en jeu sous la forme de formaline.

9. Liquide fonctionnel qui contient un mélange selon la revendication 1.

10. Liquide fonctionnel selon la revendication 9 dans lequel la quantité du mélange des composés de formules I et II représente de 0,001 à 5 % en poids du poids total du liquide.

11. En tant que liquide fonctionnel selon la revendication 9, liquide hydraulique ou liquide pour usinage de métaux qui est à base de mélanges aqueux polyglycols/éthers de polyglycols, de mélanges aqueux de glycols ou d'émulsions LH ou HL, ou antigel miscible à l'eau à base de glycols.

12. Liquide fonctionnel selon la revendication 9 dans lequel il y a un ou plusieurs co-additifs.